# EUROPEAN PATENT APPLICATION

(11) **EP 2 005 906 A2**
(43) Date of publication of application: **24.12.2008**
(21) Application number: 08011065.3
(22) Date of filing: 18.06.2008
(51) Int. Cl.: A61B 17/28, A61B 17/04, A61B 17/32

(54) **Surgical tools and operation system**

(30) Priority: 18.06.2007 JP 2007159884
(71) Applicant: Hitachi Ltd., Chiyoda-ku Tokyo 100-8280 (JP); Kyushu University, Higashi-ku Fukuoka-shi Fukuoka (JP)
(72) Inventor: Kishi, Kosuke, Chiyoda-ku Tokyo 100-8220 (JP); Hashizume, Makoto, Fukuoka-shi Fukuoka (JP); Tanoue, Kazuo, Fukuoka-shi Fukuoka (JP)
(74) Representative: Beetz & Partner

(57) **Abstract**

Provided is a surgical tools (45) having multiple functions exhibited by end effector parts (1,2,3), which is small-sized and inexpensive, and which can eliminate the necessity of replacement of surgical tools. The surgical tools incorporating: a tip end portion (5) having a plurality of end effector parts, and a tool sleeve (40) through which a transmission mechanism of driving force for applying driving force to the tip end portion is led. The plurality of end effector parts comprise a pair of a first end effector part (1) and a second end effector part (2) so as to have a first tool function, and a third end effector part (3) mated with one or both of the first end effector part and the second end effector part so as to have a second tool function. The transmission mechanism of driving force comprises a first transmission mechanism (205-280) of driving force for applying a driving force to the first end effector part and the second end effector part, and a second transmission mechanism of driving force for applying a driving force to the third end effector part, independent from the first transmission mechanism (205- 280) of driving force.

## Description

### Background of the invention

### (1) Field of the invention

The present invention relates to a surgical tools and an operating system, in particular to a surgical tools which has a plurality of surgical, functions with the use of end effector parts and which is used in an endoscopic surgical operation, and which is preferably used in an operating system.

### (2) Description of related art

There have been used various kinds of surgical tools manipulated by surgeons. The surgeons properly use various kinds of surgical tools, depending upon a case of a disease or a situation so as to carry out medical treatments. In an endoscopic surgical operation which has been carried out as Minimally invasive surgery that exerts a least load upon the patient, there has been carried out the surgical operation that a surgical tools is inserted through an outer sheath which is the so-called trocar, and which is fitted in a small hole formed in the body surface. Should the number of kinds of surgical tools be large, the operation that a surgical tools is withdrawn through the trocar, and then a different surgical tool replaced therewith is inserted through the trocar would be inevitably repeated at several times, thereby, larger time and labor are required. Further, it requires the preparation of several kinds of surgical tools, resulting in increasing of the costs.

Even in a surgical operation with the use of robots, there have caused the problems of requiring man-powers for replacements of robot surgical tool, increasing the sizes of devices for the replacement thereof or the like.

Further, these years, the technique that a surgical operation is made within an MRI system in order to carry out both diagnosis and treatment at the same time, has been noticeable. However, the image pick-up zone in the MRI system is very narrow, that is, a space through which surgical tools should be introduced into and out, is confined. Thus, it is required not only to bring the patient out from the MRI system in order to exchange surgical tool but also to exchange surgical tool with a very cramped posture within the MRI system, resulting in the requirement of much more time and manpower.

Thus, for example, JP-A-10-174689 (Patent Document 1) discloses a surgical tool which has optional functions such as electrocauterization, suctioning and injection, in addition to a basic function as a grasper-forceps.

Further, JP-A-05-184535 (Patent Document 2) also discloses the treatment technique that a plurality of surgical tools is inserted into a main catheter, being operable, independent from one another.

The surgical tools disclosed in Patent Document 1 is the one which can have a grasping function exhibited by end effector parts, and also has a plurality of additional functions such as electrocauterization, suctioning and injection, but not the one which have multiple functions by end effector parts. Thus, in the case of a treatment using various kinds of operating functions with the use of end effector parts, it is required to exchange surgical tools, and as a result, there has been the problem that the time of surgical operation correspondingly becomes longer.

Further, as to the treatment technique disclosed in Japanese Patent Document 2 using a plurality of surgical tools inserted in a single catheter, the narrower the catheter, the lager the size of a single surgical tools, relative to the catheter into which it is inserted, the surgical tools is too small to readily manipulate the surgical tools with respect to a size of an affected area, and alternatively, if a surgical tools having a size which is appropriate for an affected area were used, the catheter for receiving the surgical tools would have a larger diameter so as to require making an excessively larger hole in the surface of the human body, resulting in the problem of high infestation into the human body. Brief summary of the invention

The present invention is devised in view of the above-mentioned problems inherent to the prior art, and accordingly, one object of the present invention is to provide a surgical tools which is small-sized and which is capable of carrying out a plurality of treatment functions with the use of end effector parts without the necessity of exchange of surgical tools, thereby it is possible to aim at shortening the time of surgical operation and is also to provide an operating system using the surgical tools for minimally invasive surgery.

To the end, according to an aspect of the present invention, there is provided a surgical tools including a tip end portion having a plurality of end effector parts, and/or a tool sleeve through which a transmission mechanism of driving force is led for applying a driving force to the tip end portion, wherein the plurality of end effector parts comprising a pair of first and second end effector part having a first treatment function, a third end effector part adapted to be mated with one or both of the first and second end effector part so as to have a second treatment function, and the transmission mechanism of driving force incorporates a first transmission mechanism of driving force for applying a driving force to the first and second end effector part, and/or a second transmission mechanism of driving force for applying a driving force to the third end effector part, independent from the first transmission means.

In the specific embodiments of the first aspect of the present invention, there may be used the following configurations:
(1) The above-mentioned tip end portion comprises a housing, a first bevel gear integrally formed with the first end effector part and rotatably supported to the housing, a second bevel gear integrally formed with the second end effector part, and rotatably supported to the housing in the condition that it is laid in parallel with and opposed to the first bevel gear, a third bevel gear meshed with both first and second bevel gears and rotatably supported to the housing, a first input gear integrally formed with the third bevel gear, and/or a second input gear integrally formed with the third end effector part and rotatably supported to the housing in the condition that it is arranged in a direction crossing the first bevel gear, wherein the first input gear is coupled to the first transmission mechanism of driving force on the tool sleeve side, and/or the second input gear is coupled to the second transmission mechanism of driving force on the tool sleeve side;
(2) In the configuration (1), the first and/or second end effector part carry out opening and closing operations in response to rotation of the third bevel gear, the first bevel gear and/or the second bevel gear in association with the rotation of the first input gear, and the third end effector part carries out opening and closing operations together with one or both of the first second end effector part in association with the rotation of the second input gear;
(3) In the configuration (2), the first end effector part and/or the second end effector part exhibit a forceps function for scissoring an affected area in cooperation, and/or the first end effector part and/or the third end effector part carry out a forceps function for clamping the affected area in cooperation;
(4) In the configuration (3), there is provided a function for changing the distance between the first end effector part and/or the second end effector part to a size corresponding to a curvature of a bending needle for suture, and/or the first end effector part, the second end effector part and/or the third end effector part exhibit a function of a needle-driver for gripping the bending needle for suture;
(5) The tip end portion is flexibly supported to the tool sleeve through the intermediary of a bending joint;
(6) In the configuration (1), the tip end portion is flexibly supported to the tool sleeve through the intermediary of a bending joint, and/or the bending joint incorporates a plate for bending having a one side end part rotatably supported to the housing, and/or the other side end part rotatably supported to the tool sleeve so as to couple the housing and the tool sleeve to each other, an immovable gear fixed to the tool sleeve, and/or a guide gear fixed to the housing and adapted to be meshed with the immovable gear, the plate for bending being coupled to a third transmission mechanism of driving force of the driving force transmission power, and/or the immovable gear and/or the guide gear being configured so as to have an equal pitch circle; (7) In the configuration (6), the bending joint incorporates a first bending joint gear meshed with the first input gear which is integrally formed with the third bevel gear, and rotatably supported to the housing, a second joint gear meshed with the first bending joint gear and rotatably supported to the tool sleeve, a third bending joint gear meshed with the second input gear and rotatably supported to the housing, and/or a fourth bending joint gear meshed with the third bending joint gear and rotatably supported to the tool sleeve, the first bending joint gear and/or the third bending joint gear have pitch circles equal to that of the guide gear, and/or the second bending joint gear and/or the fourth bending joint gear have pitch circles equal to that of the immovable gear.

Further, according to the second aspect of the present invention, there is provided an operating system comprising a surgical tools for treating an affected area, and/or a tool interface for driving the surgical tools, the surgical tools having a tip end portion including a plurality of end effector parts, and/or a tool sleeve through which a drive transmission means for applying a driving force to the tip end portion is led, the tool interface incorporating a drive means for applying the driving force to the transmission mechanism of driving force, wherein the plurality of end effector parts comprises a first end effector part and/or a second end effector part in a pair for exhibiting a first tool function, and/or a third end effector part mated with one or both of the first end effector part and the second end effector part, for exhibiting a second tool function, the transmission mechanism of driving force incorporates a first transmission mechanism of driving force for applying a drive force to the first end effector part and the second end effector part, and/or a second transmission mechanism of driving force for applying a driving force to the third end effector part, independent from the first transmission mechanism of driving force, and/or the drive means incorporates a first drive means for applying a driving force to the first transmission mechanism of driving force and/or a second drive means for applying a driving force to the second transmission mechanism of driving force.

In view of the surgical tools and the operating system as stated above, there can be provided several tool functions by the end effector parts which are small-sized and inexpensive and which can eliminate the necessity of exchange of surgical tools, thereby it is possible to aim at shortening the time of minimally invasive surgery.

Further aspects, objects and advantages of the present invention will be apparent in the following description of the preferred embodiments which will be explained hereinbelow with reference to the accompanying drawings, in which:

### Brief description of the several views of the drawing

Fig. 1 is a perspective view illustrating an operating system in an embodiment of the present invention;
Fig. 2 is a front view illustrating a tip end portion shown in Fig. 1 in a condition in which all end effector parts are closed;
Fig. 3 is a front view illustrating the tip end portion shown in Fig. 2 in a condition all end effector parts are opened;
Fig. 4 is a front view illustrating the tip end portion shown in Fig. 1 in a first example in a condition in which all end effector parts are closed;
Fig. 5 is a front view illustrating the tip end part shown in Fig. 4, in a condition in which a first end effector part and a second end effector part are closed;
Fig. 6 is a front view illustrating the tip end portion in which all end effector parts are closed;
Fig. 7 is a front view illustrating the tip end portion in a condition in which the first end effector part and the second end effector part are opened, and the first end effector part and a third end effector part are opened;
Fig. 8 is a schematic view illustrating the tip end portion shown in Fig. 1 in a second example, in a condition in which a bending needle for suture is gripped by end effector parts;
Fig. 9 is a schematic view showing a condition in which a different bending needle for suture is gripped by the end effector parts shown in Fig. 2;
Fig. 10 is a detailed perspective view illustrating a surgical tools shown in Fig. 1;
Fig. 11 is an enlarged perspective view illustrating a bending joint part in the surgical tools shown in Fig. 1;
Fig. 12 is a perspective view illustrating a removal portion and a tool interface part of the surgical tools;
Fig. 13 is a perspective view illustrating the surgical tools in a condition in which it has been removed from the tool interface shown in Fig. 12, and
Fig. 14 is an enlarged exploded view illustrating a part shown in Fig. 2.

### Detailed description of the invention

Next, explanation will be made of the present invention in the form of preferable embodiments with reference to the accompanying drawings.

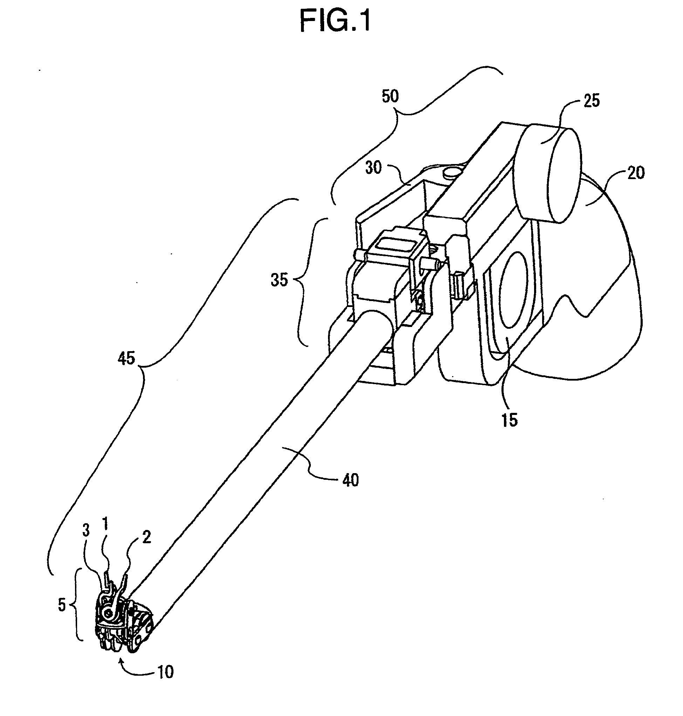

At first, explanation will be herein below made of an operating system in its entirety in an embodiment of the present invention with reference to Fig. 1 which is a perspective e view illustrating an operating system in an embodiment of the present invention. It is noted that the operating system as shown is an example of a manual operating system.

The operating system comprises a surgical tools 45 for treating an affected area, and a tool interface 50.

The surgical tools 45 incorporates a tip end portion 5 having a plurality of end effector part 1, 2, 3, a tool sleeve 40 in which a transmission mechanism of driving force for applying a driving force to the tip end portion 5 is led, a bending joint 10 for flexibly supporting the tip end portion 5 to the tool sleeve 40, and a mounting portion 35 for removably mounting the tool sleeve 40 to the tool interface 50. An end effector part 1 and an end effector part 2 for carrying out opening and closing operations are provided at the tip end of the surgical tools 45, and an end effector part 3 adapted to be operated in a plane orthogonal to the direction of movement of the end effector part 1, 2 is also provided. The tip end portion including the end effector part 1, 2, 3 can bend, relative to the tool sleeve 40 due to the provision of the bending joint 10. The surgical tools 45 is connected to the tool interface 50 through the intermediary of the mounting part 35.

The tool interface 50 incorporates a drive means for applying a driving force to the transmission mechanism of driving force for the surgical tools 45. Further, the tool interface 50 has a grip 20 to be gripped by the surgeon, a trigger 15, a dial 25 and an opening and closing flipper 30 which are to be manipulated by the surgeon. The surgeon grips the grip 20 with the palm of his hand, and manipulates the trigger 15, the dial 25 and the opening and closing flipper 30 in order to operate the bending joint 10 of the surgical tools 45 or the end effector part 1, 2, 3.

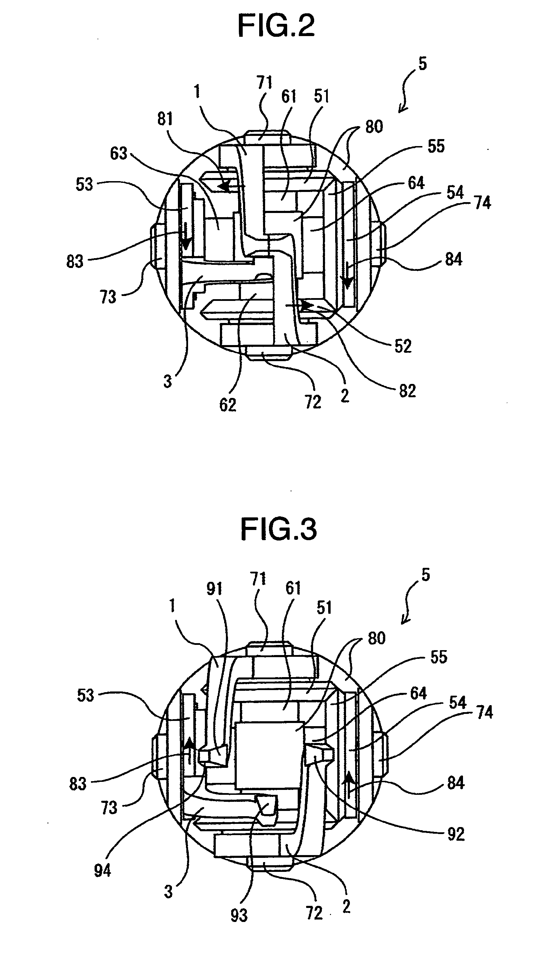

Next, detailed explanation will be made of the tip end portion 5 with reference to Figs. 2 and 3. Fig. 2 is a front view illustrating the tip end portion shown in Fig. 1, in a condition in which all end effector parts are closed, and Fig. 3 is a front view illustrating the tip end portion shown in Fig. 2, in a condition that all end effector parts are closed.

The end effector part 1, 2, 3 consist of a pair of a end effector parts (first end effector part) 1 and a end effector parts (second end effector part) 2, which exhibit a first tool function, and a end effector part 3 (third end effector part) which is mated with one or both of the end effector part 1, 2 so as to exhibit a second tool function.

The tip end portion 5 incorporates a housing 80, end effector part 1, 2, 3, a bevel gear 51 (first bevel gear) integrally formed with the end effector part 1 and rotatably supported to the housing, a bevel gear 52 (second bevel gear) integrally formed with the end effector part 2 and rotatably supported to the housing 80, being in parallel with and opposed to the bevel gear 51, a bevel gear 55 (third bevel gear) rotatably supported to the housing 80, being meshed with the bevel gears 51,52, an input gear 53 (second input gear) integrally formed with the end effector part 3 and rotatably supported to the housing 80, being arranged in a direction crossing the bevel gear 51 or the bevel gear 52.

In other words, the end effector part 1 integrally formed with the bevel gear 51, is rotatably supported to the housing 80 by means of a pin 71 together with a collar 61. The end effector part 2 integrally formed with the bevel gear 52 is rotatably supported to the housing 80 by means of a pin 72 together with a collar 62. The end effector part 3 integrally formed with the input gear 53 is rotatably supported to the housing by means of a pin 73 together with a collar 63. An input gear 54 (first input gear) integrally formed with the bevel gear 55 is rotatably supported to the housing 80 by means of a pin 74 together with a collar 64.

The bevel gear 55 is meshed with the bevel gears 51, 52, and is rotated in response to the rotation of the input gear 54, and accordingly, the bevel gears 51, 52 are rotated in association with rotation of the bevel gear 55. Thus, the rotation of the input gear 54 in the direction indicated by the arrow 84 shown in Fig. 2, causes the end effector part 1, 2 integrally formed respectively with the bevel gears 51, 52 are moved respectively in the directions indicated by the arrows 81, 82 shown in Fig. 2, resulting in postures of the end effector part 1, 2 shown in Fig. 3. Meanwhile, the end effector part 3 shown in Fig. 2 is moved as shown in Fig. 3 in association with the input gear 53 which is moved in the direction indicated by the arrow 83. On the contrary, the movements of the input gears 53, 54 as indicted by the arrows 83, 84 shown in Fig. 3 cause the end effector part 1, 2, 3 shown in Fig. 3 to be moved in a condition as shown in Fig. 2.

That is, the end effector part 1, 2 are moved at the same time in association with the motion of the input gear 54 so as to carry out opening and closing operations, but the end effector part 3 is operated by the motion of the input gear 53, independent from the motions of the end effector part 1, 2. As understood from the above-mentioned operations, the end effector part 3 is operated on a plane orthogonal to planes on which the end effector part 1, 2 are operated.

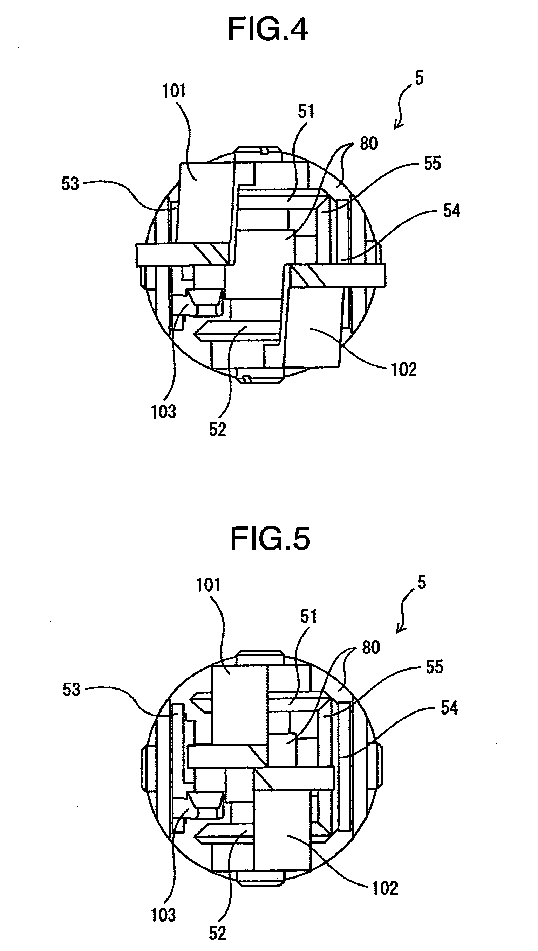

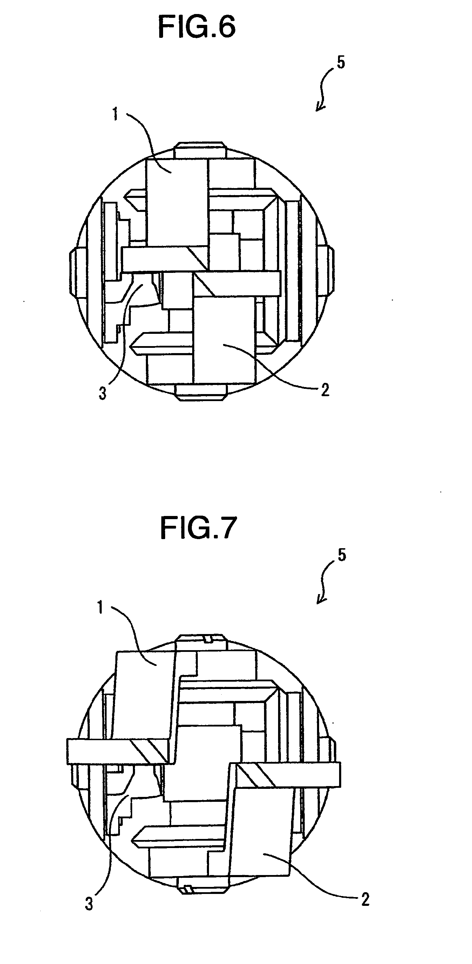

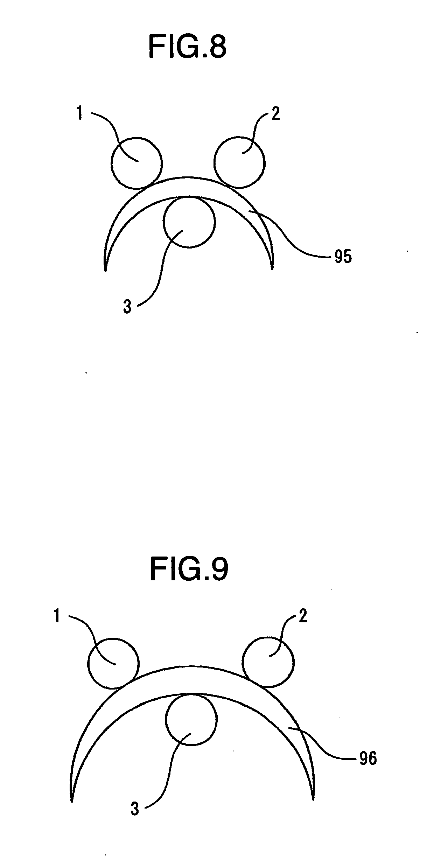

The parts of the end effector parts which define the surfaces 93, 90 and 91, 92 where the end effector parts are made into contact with each other are configured so as to have any of various shapes in order to materialize surgical tools in any of various combinations such as an scissoring forceps and a grasper-forceps, a needle-driver and a grasper-forceps or the like, that is, there can be presented a single surgical tools 45 having two tool functions, thereby it is possible to reduce the number of exchanges of the surgical tools 45 during an surgical operation with the use of an endoscope. The present invention will be hereinbelow detailed in the form of a first example shown in Figs. 4 to 7, and in the form of a second example shown in Figs. 8 and 9.

The first example shown in Figs. 4 to 7, the end effector part 1, 2 serve as scissoring end effector part 101, 102 while the end effector part 3 serves as a grasper-forceps end effector part 103. Referring to Fig. 4, the end effector part 101, 102, 103 are all opened. The scissoring end effector part 101, 102 are closed from the condition shown in Fig. 4 into a condition shown in Fig. 5. The end effector part 101, 102 can serve as a scissoring tool for scissoring an object, irrespective of the grasper-forceps end effector part 103. Meanwhile, the grasper-forceps end effector part 103 serves as a one side-opened grasper-forceps in cooperation with a side surface of the scissoring end effector part 101, for clamping the object.

Further, in the example 2 shown in Figs. 8 and 9, the end effector part 1, 2, 3 are all used so as to serve as a needle-driver. A bending needle for suture 95 shown in Fig. 8 and a bending needle for suture 96 shown in Fig. 9 have different sizes, respectively. In this case, the end effector part 1, 2 are opened by a degree which is adjusted to curvatures and sizes of the bending needle for sutures 95, 96 so as to firmly clamp the bending needle for sutures between the end effector part 1, 2 and the end effector part 3, thereby it is possible to clamp the bending needle for sutures 95, 96 having different sizes. Since the opening degree between the end effector part 1, 2 can be easily changed, the surgical operation using the bending needle for sutures 95, 96 can be carried out, irrespective of the sizes and thicknesses of the bending needle for sutures.

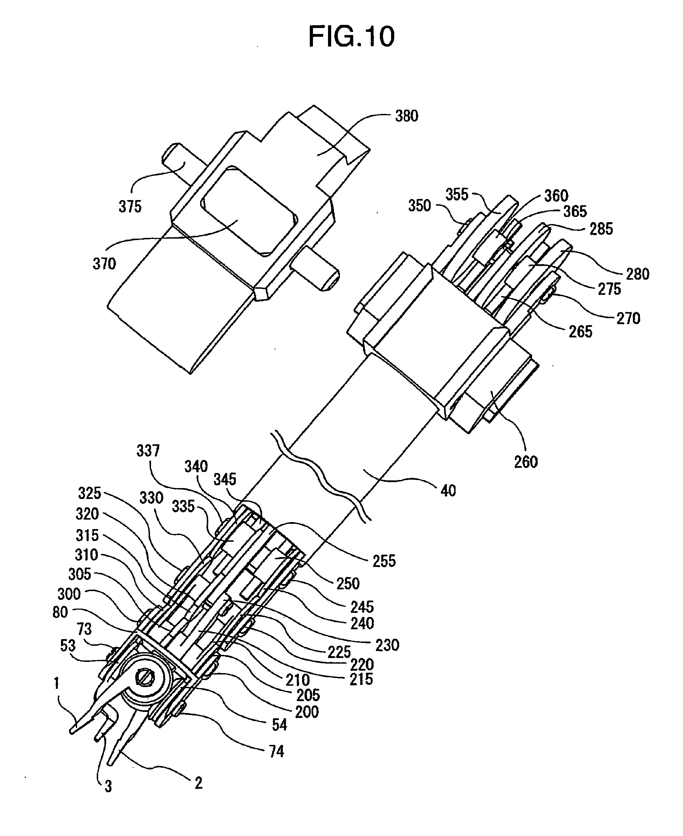

Next, detailed explanation will be made of the surgical tools 45 with reference to Fig. 10 which is a perspective view illustrating the details of the surgical tools shown in Fig. 1.

Referring to Fig. 10 in which a cover 380 is removed in order to show the interior of the surgical tools, the cover 380 is normally fixed to a tool base 260 by means of screws which are not shown. A gear retainer 370 is integrally formed with a shaft 375, and is arranged so as to be vertically movable relative to the cover 380. When the shaft 375 is displaced downward, gears 280, 285 and 355 are made into contact with the gear retainer 370 so as to be locked immovably. When the shaft 375 is displaced upward, the gears 280, 285, 355 are unlocked so as to be movable freely.

At first, explanation will be made of a mechanism for driving the input gear 54 which moves the end effector part 1, 2 or 101, 102.

The tool base 260 is mounted thereon with the gear 280 by means of pins 270, 350 so as to be rotatable. The gear 280 is integrally formed with a pinion 275 which is meshed with a rack 265. The rack 265 which extends through the tool base 260 and the tool sleeve 40, is meshed with a pinion 250 which is rotatably supported to the end of the tool sleeve 40. The pinion 250 is integrally formed with a gear 240 which is meshed with a bending joint gear 225 (second bending joint gear) that is in turn meshed with a bending joint gear 205 (first bending joint gear). The bending joint gear 205 is meshed with the input gear 54. It is noted that the mechanism from the gear 280 to the bending joint gear 205 constitutes a first transmission mechanics of driving force for applying a driving force to the end effector part 1, 2.

With this configuration, when the gear 280 is rotated together with the pinion 275, the rack 265 meshed with the pinion 275 is rectilinearly moved in the axial direction of the tool sleeve 40. The rack 265 rotates the pinion 250 in the end of the tool sleeve 40, and accordingly, the gear 240 integrally formed with the pinion 250 is rotated. The rotation of the gear 240 causes the bending joint gear 225 meshed with the gear 240 to rotate, and accordingly, the bending joint gear 205 meshed with the bending joint gear 225 is rotated. The rotation of the bending joint gear 205 causes the input gear 54 meshed with the bending joint gear 205 to rotate, and as a result, the end effector part 1, 2 or 101 or 1,02 are operated as stated above.

Next, explanation will be made of the mechanism for driving the input gear 53 which moves the end effector part 3 or 103 as stated above.

A gear 355 is mounted to the tool base 260 by means of pins 270, 350 so as to be rotatable. The gear 355 is integrally formed with a pinion 360 which is meshed with a rack 345 that extends through the tool base 260 and the tool sleeve 40, and is then meshed with a pinion 335 rotatably supported to the end of the tool sleeve 40. The pinion 335 is integrally formed with a gear 340 which is in turn meshed with a bending joint gear 330 (fourth bending joint gear) that is meshed with a bending joint gear 310 (third bending joint gear). The bending joint gear 310 is meshed with the input gear 53. It is noted that the mechanism from the gear 355 to the bending joint gear 310 constitute a second transmission mechanism of driving force for applying a driving force to the end effector part 3.

With the above-mentioned configuration, when the gear 355 is rotated together with the pinion 360, the rack 345 meshed with the pinion 360 is rectilinearly moved in the axial direction of the tool sleeve 40. The rack 345 rotates the pinion 335 in the end of the tool sleeve 40, and accordingly, the gear 340 which is moved integral with the pinion 335 is rotated. The rotation of the gear 340 causes the bending joint gear 310 meshed with the bending joint gear 330 to rotate. The rotation of the bending joint gear 310 causes the input gear 53 meshed with the bending joint gear 310 to rotate, and as a result, the end effector part 3 or 103 is operated as stated above.

It is noted that the pinion 250 and the gear 240 moved integral with the pinion 250, and the pinion 335 and the gear 340 moved integral with the pinion 335 are rotatably supported to the end of the tool sleeve 40 by means of pins 245, 337. The bending joint gear 225 and the bending joint gear 330 are rotatably supported to the end of the tool sleeve 40 by means of pins 220, 325.

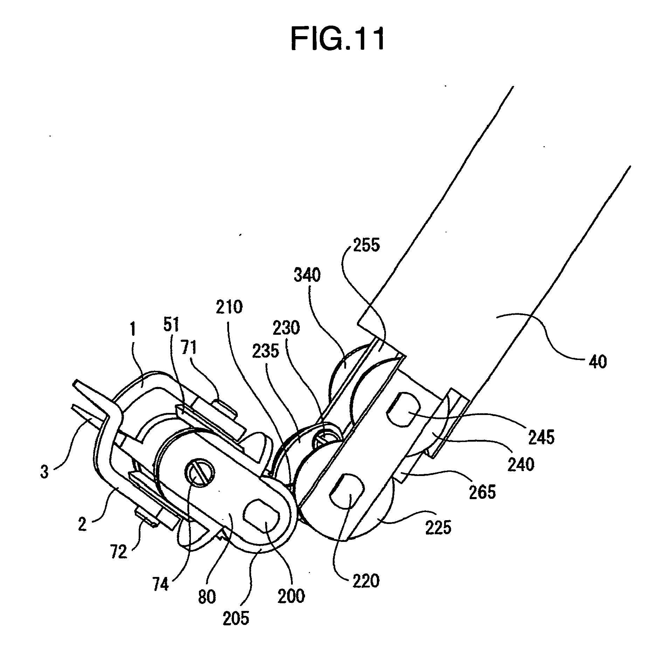

Next, explanation will be made of the bending motion of the surgical tools 45 with reference to Figs. 10 and 11 which is an enlarged perspective view illustrating the bending joint portion of the surgical tools shown in Fig. 1. The surgical tools 45 is configured so as to enable the tip end portion 5 to bend, relative to the tool sleeve 40.

In the tip end part of the surgical tools 40, the immovable gear 315 is secured by pins 325, 220 and pins 245 and 340. The immovable gear 315 is adapted to be meshed with a guide gear 305 fixed to the housing 80.

It is noted that the bending joint gear 225, the immovable gear 315 and the bending joint gear 330 have gear pitch circles which are equal to one another, and further, the bending joint gear 205, the guide gear 305 and the bending joint gear 310 have gear pitch circles which are equal to one another. Further, the bending joint gear 205 and the bending joint gear 310 are rotatably supported to the housing 80 by means of pins 200, 300.

The tool sleeve 40 bend to the housing 80, and are arranged so as to maintain the space therebetween by a predetermined distance by means of the immovable gear 315 and the guide gear part 305 which are meshed with each other, and are connected to each other through the intermediary of distance holding plates 210, 320 which are rotatable with respect to the pin 220 and the pin 325, and the pin 200 and the pin 300.

The gear 285 is connected to a plate for bending 215 through the intermediary of a link 225 which is rotatably supported by a pin 365. Further, the plate for bending 215 is rotatably connected to the link 255 by means of a pin 230. The plate for bending 215, the links 255, the gear 355, the tool sleeve 40 and the tool base 260 are configured, having a parallel link relationship. When the gear 285 is swung around the pins 270, 350 as an axis, the plate for bending 215 is swung around the pins 220, 325 as an axis. Since the plate for bending 215 is connected to the housing 80 through the intermediary of the pins 200, 300, the plate for bending 215 which is moved around the pins 220, 324 as a center, a force for moving the housing is transmitted. At this time, since the guide gear 305 of the hosing 80 is meshed with the immovable gear 310, the housing 80 is rotated by this force in its entirely around the immovable gear 315. At this time, since the bending joint gear 310 and the bending joint gear 330, and the bending joint gear 205 and the bending joint gear 225 are moved with a relationship therebetween similar to that between the guide gear 305 and the immovable gear 315, the flexible gear 310 and the flexible gear 205 are not moved relative to the housing 80. Thus, irrespective of the motion of the bending joint gear 10, the end effector parts can be operated, independent from one another. It is noted that the mechanism of the gear 285 and the link 225 constitutes a third transmission mechanism of driving force for applying a driving force to the plate for bending 215.

As understood from the configuration as stated above, the surgical tools 45 in this embodiment can be operated without using any wire. Since the end effector parts can be operated with no use of a wire, the maintainability of the surgical tools 45 can be enhanced, and further, the control ability thereof can be enhanced without occurrence of a problem of loosening of a wire, a problem of cooperative control or the like.

However, it is noted that the surgical tools having the multiple functions can be materialized by using wires so as to operate the gear 240 or the gear 340 and the plate for bending 215 without using the rack 265, the rack 345 and the link 255.

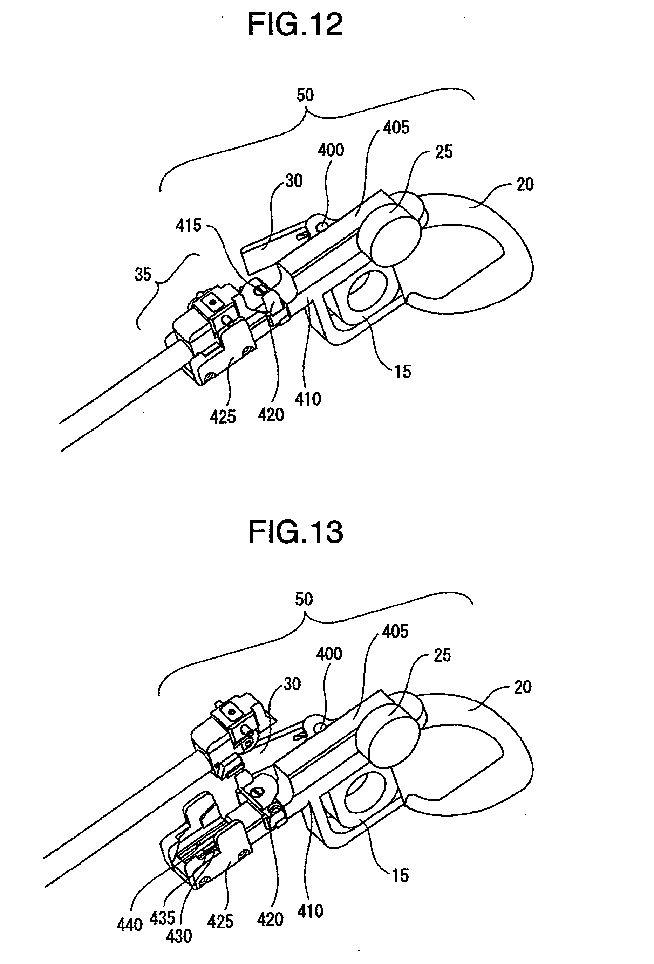

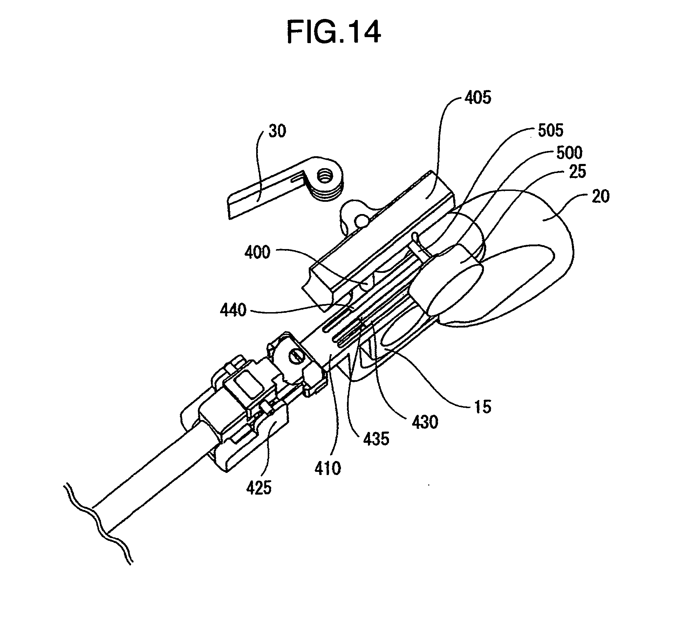

Next, referring to Figs. 12 to 14, detailed explanation will be made of the tool mounting portion 35 and the tool interface 50. Fig. 12 is a perspective view illustrating the removal portion and the tool interface of the surgical tools shown in Fig. 1, Fig. 13 is a perspective view illustrating the tool interface 50 from which the surgical tools 45 shown in Fig. 12 is removed, and Fig. 14 is an exploded enlarged view illustrating a part shown in Fig. 12.

The removal portion 35 of the surgical tools 45 is attached to the tool interface 50 along a guide part of a tool guide 425 of the tool interface 50 as shown in Fig. 12, and is locked thereto by a tool mounting lever 420. The tool mounting lever 420 is rotatably mounted to a guide base 410 by means of a pin 415 serving as a shaft, and accordingly, by manipulating the tool mounting lever 420, the surgical tools 45 can be removed from and attached to the tool interface 50.

The guide base 410 is mounted thereon with racks 430, 435, 440 which can be therefore rectilinearly moved as shown in Fig. 13. These racks 430, 435, 440 are adapted to be meshed, respectively with the gears 280, 285, 355 shown in Fig. 10 after the surgical tools 45 is attached, and accordingly, by moving the racks 430, 435, 440, the end effector part 1, 2, 3 at the tip end of the tool can be operated.

The mechanism for moving the racks 430, 435, 440 is shown in Fig. 14 in which a guide cover 405, a flipper 30 and the like are disassembled for the brevity of explanation.

The rack 430 is meshed with a dial 25 and a pinion 500 which is moved integral with a shaft 505 which is rotatably mounted to the guide cover 405. In the case that the surgeon grips the grip 20 with his right hand, by turning the dial 25 with his thumb, the rack 430 is rectilinearly moved, and accordingly, the end effector part 3 at the tip end of the surgical tools is operated through the intermediary of a gear train.

Further, the rack 435 is coupled with the trigger 15, and accordingly, it can be rectilinearly moved, relative to the guide base 410. In the case that the surgeon grips the grip 20 with his right hand, he inserts his forefinger or middle finger in a hole in the trigger 15 in order to push and pull the trigger 15, and accordingly, the rack 435 is rectilinearly moved so that the tip end portion 5 bends around the bending joint 10.

Further, the rack 440 is meshed with the shaft 400 integrally formed with the pinion. The shaft 400 is secured to the flipper 30, and is rotatably attached to the guide cover 405 and the guide base 410. In the case that the surgeon grips the grip with his right hand, by pushing the flipper 30 with his forefinger, the pinion of the shaft 400 integrally formed with the flipper 30 rectilinearly moves the rack 440 so as to cause the end effector part 1, 2 at the tip end of the surgical tools to carry out closing operation. When the flipper 30 is opened by a spring which is not shown, the end effector part 1, 2 are also opened.

As understood from the explanation mentioned above, the operating directions of the end effector part 1, 2, 3 at the tip end of the surgical tools, and the moving directions of the tool interface 50 manipulated by the surgeon fall in one and the same plane. Since the moving directions of the flipper 30 and the operating directions of the end effector part 1, 2, the rotating direction of the dial and the operating direction of the end effector part 3, and the plane in which the trigger 15 is moved and the plane in which the bending joint 10 is moved, are the same, thereby it is possible to provide a surgical tools which is intuitive and which can be easily manipulated.

Further, by changing the operating direction of the tool interface 50 into any of its respective directions, intuitive and easy manipulation can be made even though multiple functions are effected at the tip end of the surgical tools.

It is noted that the surgical tools 45 and the tool interface 50 can be easily disconnected from each other, and accordingly, even though several kinds of surgical tools each having two kinds of functions are prepared by a number required for a specific surgical operation, no more than one tool interface 50 is required, and accordingly, its can offer an advantage of cost reduction. The present invention is of course applicable even though the surgical tools and the tool interface are not disconnectable from each other.

Further, it is noted that the surgical tools 45 can be used being coupled not only to the tool interface 50 but also to a manipulator.

According to the present invention, the motions of the end effector part 1, 2, 3 capable of carrying out two kinds of surgical operating functions, are orthogonal to one another, and accordingly, they do not interfere with one another. Further, the object can be accessed with the surgical tools 45 with any of different operating functions only by turning the shaft of the surgical tools without changing the position thereof, and accordingly the time of surgical operation can be saved. Further, since the single surgical tools 45 can have multiple operating functions, the number of surgical tools to be prepared can be reduced, resulting in cost reduction.

In the surgical tools apparatus according to the present embodiment, the tip end part of the surgical tools can be bended, independent from the end effector part 1, 2, 3 provided at the tip end part of the surgical tools. It can be provided as a manual surgical operating unit without using electronic control with an actuator or the like, and the manual control portion thereof can control the motions of the end effector parts at the tip end of the surgical tools, thereby it is possible to intuitively manipulate the surgical tools.

Further, this surgical tools 45 can be disconnected from the manual control input portion, and accordingly, the same surgical tools can be attached to a manipulator. Thereby it is possible to operate the surgical tools by means of the manipulator.

Further, this surgical tools 45 having the three end effector part 1, 2, 3 at the tip end thereof can have two operating functions with the end effector parts which have sizes equal to that of those of conventional surgical tools, or can grips any of bending needle for sutures, irrespective of sizes of the needles. Further, since the operating planes of the end effector parts are coincident with the input operating planes at the manipulating interface, the manipulation thereof can be simplified, thereby it is possible to carry out intuitive manipulation with less occurrence of erroneous operation. Thus, the time required for exchanging surgical tools through the trocar can be saved, and thereby it is possible to exhibit several advantages that the time of a surgical operation is shortened, the surgical operation is hygienic and safe, the number of surgical tools required for the surgical operation can be reduced to a half of the number of conventionally used surgical tools, and the costs can be reduced. Further, this surgical tools can be driven without using any wire, and accordingly, the control ability and the maintainability can be enhanced with no possibility of occurrence of problems of loosening and breakage of a wire. Further, the bending joints can be operated, independent from one another, thereby it is possible to enhance the controllability and the safety.

It should be further understood by those skilled in the art that although the foregoing description has been made on embodiments of the invention, the invention is not limited thereto and various changes and modifications may be made without departing from the spirit of the invention and the scope of the appended claims.

Features, components and specific details of the structures of the above-described embodiments may be exchanged or combined to form further embodiments optimized for the respective application. As far as those modifications are readily apparent for an expert skilled in the art they shall be disclosed implicitly by the above description without specifying explicitly every possible combination, for the sake of conciseness of the present description.

## Claims

1. A surgical tools **characterized by** comprising:
a tip end portion (5) having a plurality of end effector parts (1,2,3), and
a tool sleeve (40) through which a transmission mechanism of driving force for applying a driving force to the tip end portion (5) is led, wherein
the plurality of end effector parts comprises a pair of a first end effector part (1) and a second end effector part (2) so as to have a first tool function, and a third end effector part (3) mated with one or both of the first end effector part (1) and the second end effector part (2) so as to have a second tool function, and
the transmission mechanism of driving force comprises a first transmission mechanism (205-280) of driving force for applying a driving force to the first end effector part (1) and the second end effector part (2), and a second transmission mechanism (310-355) of driving force for applying a driving force to the third end effector part (3), independent from the first transmission mechanism (205-280) of driving force.

2. A surgical tools as set forth in claim 1,
**characterized in that** the tip end portion (5) comprises
a housing (80),
a first bevel gear (51) integrally formed with the first end effector part (1), and rotatably supported to the housing (80),
a second bevel gear (52) integrally formed with the second end effector part (2), and rotatably supported to the housing (80), being parallel with and opposed to the first bevel gear (51),
a third bevel gear (55) rotatably supported to the housing (80), being meshed with the first and second bevel gears (51, 52),
a first input gear (54) integrally formed with the third bevel gear (55),
a second input gear (53) integrally formed with the third end effector part and rotatably supported to the housing, being arranged in a direction crossing the first bevel gear (51), the first input gear (54)is coupled with the first transmission mechanism (205-280) of driving force on the tool sleeve side, and the second input gear (53) is coupled to the second transmission mechanism (310-355) of driving force on the tool sleeve side.

3. A surgical tools as set forth in claim 1 or 2, **characterized in that** the first end effector part (1) and the second end effector part (2) carry out opening and closing operation in response to rotation of the third bevel gear (55), the first bevel gear (51) and the second bevel gear (53) in association with rotation of the first input gear (54), and the third end effector part (3) carry out opening and closing operation in cooperation with one or both of the first end effector part (1) and the second end effector part (2) in association with rotation of the second input gear (53).

4. A surgical tools as set forth in claim 3, **characterized in that** the first end effector part (1) and the second end effector part (2) exhibit a forceps function for scissoring an affected area in cooperation, and the first end effector part (1) and the third end effector part (3) exhibit a forceps function for clamping an affected area in cooperation.

5. A surgical tools as set forth in claim 3, **characterized in that** there is exhibited a function in which the distance between the first end effector part (1) and the second end effector part (2) is changed, corresponding to a curvature of a bending needle (95) for suture, and the first end effector part (1) and the second end effector part (2), and the third end effector part (3) exhibit a function of a needle-driver for gripping the bending needle (95) for suture.

6. A surgical tools according to at least one of the preceding claims, **characterized in that** the tip end portion (5) is flexibly supported to the tool sleeve (40) through the intermediary of a bending joint (10).

7. A surgical tools according to at least one of the preceding claims, **characterized in that** the tip end part (5) is flexibly supported to an end of the tool sleeve (40) through the intermediary of a bending joint (10), the bending joint comprising a plate (215) for bending having one end part which is rotatably supported to the housing (80), and the other end part which is rotatably supported to the tool sleeve (40), an immovable gear (315) secured to the tool sleeve (40) so as to couple the housing (80) with the tool sleeve (40), and a guide gear (305) secured to the housing (80) and adapted to be meshed with the immovable gear (315), the plate (215) for bending being coupled to a third transmission mechanism of driving force for the transmission mechanism of driving force, and the immovable gear (315) and the guide gear (305) having pitch circles which are equal to each other.

8. A surgical tools as set forth in claim 7, **characterized in that** the bending joint (10) comprises a first bending joint gear (205) meshed with the first input gear (54) integrally formed with the third bevel gear (55) and rotatably supported to the housing (80), a second bending joint gear (225) meshed with the first bending joint gear (205) and rotatably supported to the tool sleeve (40), a third bending joint gear (310) meshed with the second input gear (53) and rotatably supported to the housing (80), and a fourth bending joint gear (330) meshed with the third bending joint gear (310) and rotatably supported to the tool sleeve (40), the first bending joint gear (205)and the third bending joint gear (310) having a pitch circle equal to that of the guide gear (305), the second bending joint gear (225) and the fourth bending joint gear (330) having a pitch circle equal to that of the immovable gear (315).

9. An operation system comprising a surgical tools (45) for treating an affected area, and a tool interface (50) for driving the surgical tools (45),
the surgical tools (45) incorporating a tip end portion (5) having a plurality of end effector parts (1,2,3), and a tool sleeve (40) through which a transmission mechanism of driving force for applying a driving force to the tip end portion (5) is led, and
the interface (50) incorporating a drive means for applying a driving force to the transmission mechanism of driving force,
**characterized in that**
the plurality of end effector parts comprises a pair of a first end effector part (1) and a second end effector part (2) so as to have a first tool function, and a third end effector part (3) mated with one or both of the first end effector part (1) and the second end effector part (2) so as to have a second tool function,
the transmission mechanism of driving force comprises a first transmission mechanism (205-280) of driving force for applying a driving force to the first end effector part (1) and the second end effector part (2), and a second transmission mechanism (310-355) of driving force for applying a driving force to the third end effector part (3), independent from the first transmission mechanism (205-280)of driving force, and
the drive means comprises a first drive means for applying a driving force to the first transmission mechanism (205-280) of driving force, and a second drive means for applying a driving force to the second transmission mechanism (310-355) of driving force, independent from the first drive means.
